# FASCICULE DE BREVET EUROPEEN

(11) **EP 0 696 163 B1**
(45) Date de publication et mention de la délivrance du brevet: **05.08.1998**
(21) Numéro de dépôt: 94915180.7
(22) Date de dépôt: 22.04.1994
(51) Int. Cl.: A01H 4/00

(54) **SEMENCE ARTIFICIELLE COMPRENANT UN TISSU MERISTEMATIQUE ENROBE**
KÜNSTLICHES SAATGUT MIT UMHÜLLTEM MERISTEMGEWEBE
ARTIFICIAL SEED CONTAINING COATED MERISTEMATIC TISSUE

(30) Priorité: 27.04.1993 FR 9305192
(43) Date de publication de la demande: 14.02.1996
(73) Titulaire: RHONE-POULENC AGRO, 69009 Lyon (FR)
(72) Inventeur: DUPUIS, Jean-Marc, F-69009 Lyon (FR); MOLLE, Francis, F-69100 Villeurbanne (FR)
(74) Mandataire: Chretien, François
(86) Numéro de dépôt international: FR9400457
(87) Numéro de publication internationale: WO9424847

(56) Documents cités:
- WO-A-87/04044
- WO-A-92/07457
- WO-A-92/10087
- US-A- 4 769 945
- US-A- 4 808 430

## Description

La présente invention concerne un tissu méristématique comprenant un tissu méristematique et un support solide.

En particulier la demande WO/FR 92/10087 concerne une semence artificielle contenant un tissu méristématique en contact avec un support solide sec ou sèchable, ces deux éléments étant entourés d'une enveloppe polymérique hydrosoluble, elle même recouverte sur sa partie intérieure d'une couche étanche. Ce type de semence artificielle présente l'inconvénient d'être non déshydratable et de ne pouvoir se conserver dans une ambiance à humidité relative supérieure à 50%.

On sait que dans les années récentes, on s'est intéressé de plus en plus aux tissus méristématiques, ceux-ci permettant de mettre à la disposition des utilisateurs des semences artificielles comportant des caractéristiques générales homogènes et des caractéristiques génétiques particulières bien définies, précises, sophistiquées, déterminées et identifiées, en vue d'obtenir des variétés végétales bien déterminées. On a proposé des systémes d'enrobage divers mais qui ne donnent pas de taux satisfaisant de germination.

Un but de la présente invention est de fournir un tissu méristématique enrobé perfèctionné, n'ayant pas les inconvénients des tissus méristématiques connus.

Il a été maintenant été trouvé que ces buts pouvaient être atteints en tout ou partie grâce aux tissus méristématiques enrobés selon l'invention.

L'invention a pour objet une semence artificielle comprenant un tissu méristématique enrobé, laquelle comprend un tissu méristématique, sec ou séchable, et un support solide sec ou séchable, capable d'absorber un milieu nutritif, en contact avec le tissu méristématique, le recouvrant partiellement, de sorte que le tissu méristématique reste en contact avec un volume d'air, et ne faisant pas obstacle à la germination, caractérisée en ce que le support comporte une cavité de volume supérieur à celui du tissu méristématique.

Par "tissu méristématique", on entend, au sens de la présente invention, tout tissu végétal, ou ensemble de cellules végétales, capable, lorsqu'il est mis dans des conditions convenables, de se développer jusqu'à former une plante entière ou partie de plante entière. Sous cette expression on inclut toute sorte de tissus végétaux, notamment: le tissu somatique(graine), l'embryon somatique, le tissu zygotique, le germe, le bourgeon adventice, les pousses, le primordium de tige (connu en anglais sous le nom de shoot primordium), les analogues de protocorn (connus en anglais sous le nom de protocorn like body), le tissu connu en anglais sous le nom de green spot, les cellules germinales et semences naturelles (connues en anglais sous le nom de germ line), les jeunes plants.

Les végétaux concernés par l'invention sont de nature les plus diverses et incluent les cultures vivrières telles que le riz, le blé, l'orge, le maïs, le soja ; les cultures légumières telles que le céleri, le persil la salade, le chou-fleur, la carotte, l'aubergine la tomate, l'oignon, l'ail, le gingembre, les fraises, les melons, l'asperge ; les cultures vivrières et/ou industrielles telles que le colza, la canne à sucre, la betterave à sucre, le tabac; les cultures à but médical telles que la belladone, le ginseng; les cultures ornementales telles que les chrysanthèmes, les glaïeuls, les lys, les orchidées, les amaryllis, les géranium, les begonia, les violettes du Cap, la poinsettia; des arbres ou espèces arborescentes ou arbustes telles que les résineux, les palmiers, les arbres fruitiers, la vigne, les arbres à feuilles caduques, et autres.

Le tissu méristématique petit être sec, déhydraté ou sous forme hydratée jusqu'à 100% d'humidité.

Comme matériau servant de support pour les tissus méristématiques, on peut citer les matériaux fibreux (tels que la laine, le coton ou la laine de verre ou la laine de roche), les matériaux poreux, les matériaux alvéolaires (tels que les mousses en polymères synthétiques, notamment les polyéthers et les polyuréthanes). Plus particulièrement on petit citer le sable, l'argile, la vermiculite, les billes de verre, le coton, le papier le son de céréales, la sciure de bois, la laine, l'acétate de cellulose et autres dérivés de cellulose, auxquels cas le plus souvent, ces matériaux doivent pour être structurés par addition d' au moins un liant et/ou d'épaississant. Ce support, qui généralement donne la forme à l'ensemble, peut être de forme quelconque mais aura de préférence une forme convenable pour une fabrication industrielle et une manipulation facile par l'utilisateur. Elle pourrra par exemple être cubique parallépipédique, cylindrique, semi - cylindrique, sphérique mais d'autres formes ne sont pas exclues. Elle est telle qu'un de ses côtés puisse porter ou enserrer le tissu méristématique; elle comporte une cavité de volume supérieur à celui du tissu méristématique laissant ainsi à ce dernier un volume d'air fournissant l'oxygène nécéssaire à la germination ou au développement.

Le support peut être sec, déhydraté ou sous forme hydratée jusqu'à 100% d'humidité.

Selon l'invention, le tissu méristématique est inséré dans le support, sans que celui-ci le recouvre complètement de manière qu'il y soit toujours en contact avec un certain volume d'air.

De manière préférée, le tissu méristématique enrobé selon l'invention comprend en outre, autour du support, au moins une couche de pellicullage le recouvrant partiellement, de sorte que le tissu méristématique reste en contact avec un volume d'air.

Par pelliculage au sens de la présente invention on entend une pellicule ne modifiant sensiblement pas la forme du support, d'épaisseur très régulière, généralement comprise entre 1 et 250 microns, de préférence entre 5 et 100 microns. Cette pellicule est constituée essentiellement d'une substance filmogène ayant par nature des propriétés d'hydrophobie et/ou d'imperméabilité à l'eau liquide et ne faisant pas obstacle à la sortie des racines.

Comme substance filmogène on peut citer les polymères synthétiques, polymères naturels, les polymères artificiels, les substances filmogènes non polymèriques.

Comme exemples de polymères synthétiques utilisables dans l'invention on peut citer les résines vinyliques, notamment le polyéthylène, le polypropylène, le polystyrène, le polyméthacrylate de méthyle, le polychlorure de vinyle, le polyfluorure de vinylidène, le polyfluorure de vinyle, le polychlorure de vinylidène, le polychlorotrifluoro éthylène, le polytéréphtalate d'éthylène glycol, le polyacétate de vinyle, les copolymères éthylene-esters vinyliques ; les polymères vinyliques à base de formaldéhyde ou butyraldéhyde; les polyesters notamment le polytéréphtalate d'éthylène glycol; les polycarbonates; les polyamides, notamment le poly(11-aminoundécanamide) ou nylon 11; les polyéthers, notamment les polyoxyde de phénylène; le polychloroprène, le polyisoprène, les polyuréthane, le caoutchouc butyl, les silicones, notamment les polymères organopolysiloxaniques.

Comme exemples de polymères naturels on peut citer le gutta percha, le caoutchouc naturel.

Comme exemple de polymères artiticiels on peut citer les dérivés imperméables à l'eau de la cellulose, notamment l'éthylcellulose, l'acétate de cellulose, le propionate de cellulose, la nitrocellulose.

Comme exemple de substances filmogènes non polymériques on peut citer les graisses et les cires, notamment l'huile de palme et l'huile de coprah.

La réalisation de la pellicule étanche à l'eau à l'extérieur du support du tissu méristématique petit être effectuée par les diverses techniques connues en soi, de préférence par pulvérisation, badigeonnage ou trempage. Cette application peut mettre en oeuvre des solutions, des émulsions ou des suspensions.

Outre les divers constituants déjà décrits, les tissus méristématiques selon l'invention peuvent contenir encore divers autres types d'additifs ou d'adjuvants, notamment des produits agrochimiques tels que insecticides, fongicides, bactéricides, des engrais, des oligoéléments, des éléments nutritifs ou des additifs de présentation tels que, charges colorants ou adhésifs.

Le tissu méristématique enrobé selon l'invention ainsi décrit présente des propriétés remarquables et améliorées par rapport aux techniques antérieures, à savoir une bonne aptitude à la dessication et qu'il assure non seulement la survie du tissu végétal mais petit redonner (conversion), après réhydratation, à une plante entière avec un rendement remarquable correspondant aux besoins de l'agriculture moderne.

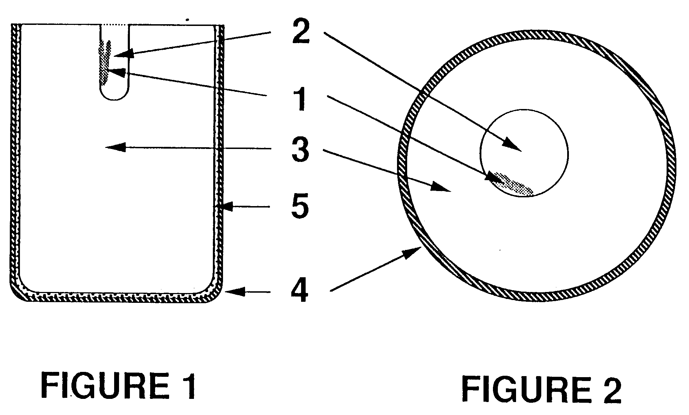

L'invention sera mieux comprise à l'aide des figures 1 et 2 annexées données à titre d'illustration non limitative.

La figure 1 représente un mode de réalisation de tissu méristématique enrobé, de forme, sensiblement cylindrique, dans laquelle un tissu méristématique, ici un embryon somatique 1 est disposé dans une cavité intérieure 2 pratiquée dans un support solide 3, entouré d'une couche externe 4 de pelliculage, et entre le support 2 et la couche de pelliculage 4, se trouve une couche d'enrobage 5, les deux couches 4 et 5 ne couvrant pas le dessus du support; celui-ci est une portion

cylindrique de filtre en cellulose. La figure 2 représente une coupe transversale d'un autre mode de réalisation de tissu méristématique enrobé selon l'invention, de forme sensiblement sphérique, ayant les mêmes constituants qu'à la figure 1, si ce n'est que la cavité 2 ne pas débouche vers l'extérieur.
L'invention a également pour objet un procédé d'obtention du tissu méristématique enrobé selon l'invention, dans lequel on cultive une suspension cellulaire végétale jusqu'au stade embryonnaire créant un tissu méristématique, et on fixe le tissu méristématique à un support solide sec ou séchable, capable d'absorber un milieu nutritif et permettant sa germination, de sorte que le support le recouvre partiellement et que le tissu méristématique reste en contact avec un volume d'air, caractérisé en ce que le support comporte une cavité de volume supérieur à celui du tissu meristématique.

Dans un première étape en soi connue, on produit des tissus mérismatiques selon un méthode en soi connue de production de masse, par exemple selon F.Molle et al, Synseeds. Ch 15, p. 257 -287, par embryogénèse, éventuellement récolte et maturation sur un milieu contenant du sucrose, soit un milieu solide gélosé à une température d'environ 4°C, soit un milieu liquide à une température de 20-25°C. Si la récolte des embryons n'a pas encore été faite elle peut l'être à ce moment.

Les tissus mérismatiques sont peuvent être ensuite soumis à une dessication dans une enceinte déssèchante, de manière à obtenir des tissus ayant une teneur en eau faible, de préférence inférieure à 0,40 g par gramme de poids sec.

Le tissu méristématique ainsi obtenu est disposé, fixé, éventuellement inséré, avant ou après pelliculage et/ou enrobage sur une face d'un support tel que défini ci-dessus, de préférence en un point central ou dans une cavité ménagée dans celle ci, de manière qu'il y ait un volume d'air au voisinage du tissu méristématique.

De préférence, afin d'assurer une étanchéité vis à vis du milieu de culture extérieur, le support portant éventuellement le tissu méristématique est recouvert d'abord d'une couche d'enrobage à base de matériaux tels que l'argile, avantageusement avec un liant et/ou un épaississant, qui après séchage, forme une sorte de coque dont le rôle est d'éviter que les polymères en solution, utilisées pour le pelliculage ne pénètrent au coeur du support, bouchant ainsi ce dernier et freinant, voire inhibant, le développement de la germination. On applique ensuite une solution aqueuse à base d'un polymère filmogène qui par séchage donne une couche de pelliculage.

Pour leur emploi dans la pratique, par semis ou mise en culture quelconque, les tissus méristématiques enrobés selon l'invention, s'ils sont dans un état déshydraté, peuvent être soumis à une réhydratation progressive.

Le contrôle de la capacité à la dessication des tissus mérismatiques ainsi que celui de la qualité de germination permettent de montrer les excellentes qualités des produits selon l'invention, à savoir leur excellente aptitude à la dessication et et de conservation avec maintien de leurs capacités de germination.

L'invention sera mieux comprise à l'aide des exemples de réalisation donnés à titre d'illustration non limitative.

### EXEMPLE 1 : préparation des embryons somatiques :

Le matériel végétal est issu d'une "lignée" mâle stérile de carotte (souche C4). Les graines, fournies par la société Tézier, sont mises à germer aseptiquement. Les hypocotyles sont découpés et placés sur milieu MS (Murashige et Skoog)H⁺. de composition indiquée au tableau 1 ci-après, caractérisé par la présence d'une auxine, l'acide 2,4-dichlorophénoxyacétique, en vue d'obtenir des cals. Les cals sont ensuite placés en milieu liquide MSH⁺ afin d'obtenir une suspension cellulaire indifférenciée. La suspension cellulaire est entretenue sur milieu MSH⁺ dans des Erlenmeyers de 250 ml contenant 100 ml de milieu. Le repiquage de la suspension s'effectue tous les 12 jours par filtration sur toile a bluter de 50 µ de vide de maille (Scrynel; Suisse). La fraction retenue par le filtre est pesée et mise en suspension dans des Erlenmeyers à raison de 1 g de biomasse pour 100 ml de milieu. Cette étape peut être effectuée dans des Erlenmeyers de 250 ml contenant 100 ml de milieu MSH⁺ (phase d'entretien) ou dans des Erlenmeyers de 1 litre contenant 500 ml de milieu (phase de multiplication). La suspension est maintenue en chambre de culture à 25°C sous une photopériode de 16 h de jour et 8 h de nuit et une intensité lumineuse de 15 µE.m⁻².s^{-1.}Elle est maintenue en agitation à 100 tours.min⁻¹ sur agitateur orbital (agitateur Biobloc scientific 74403).

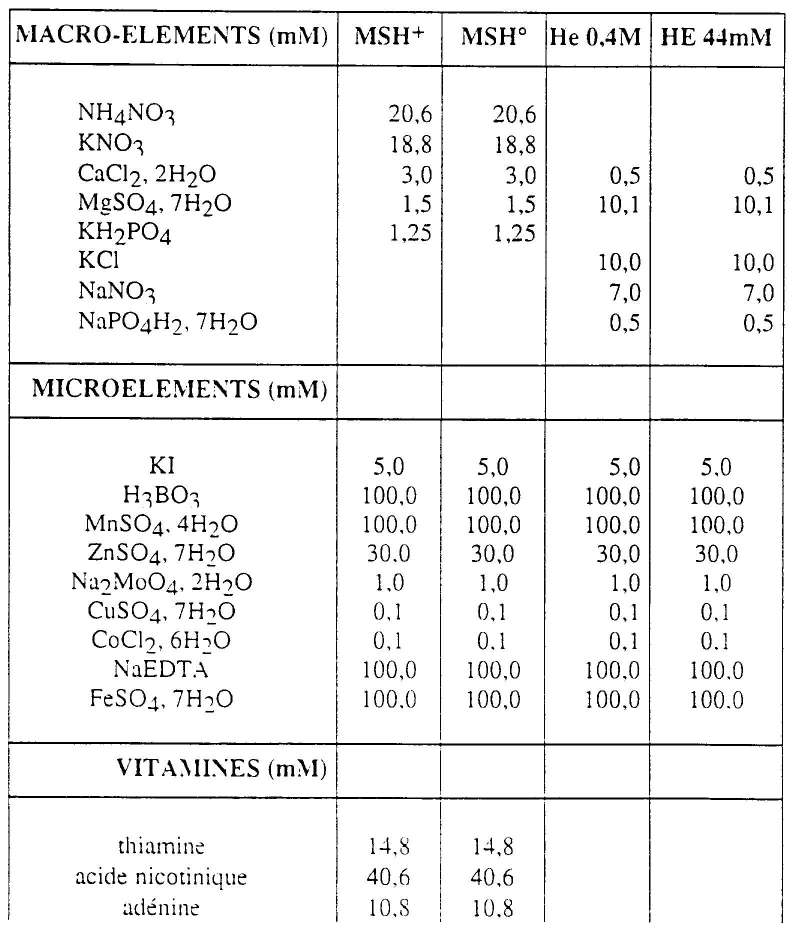

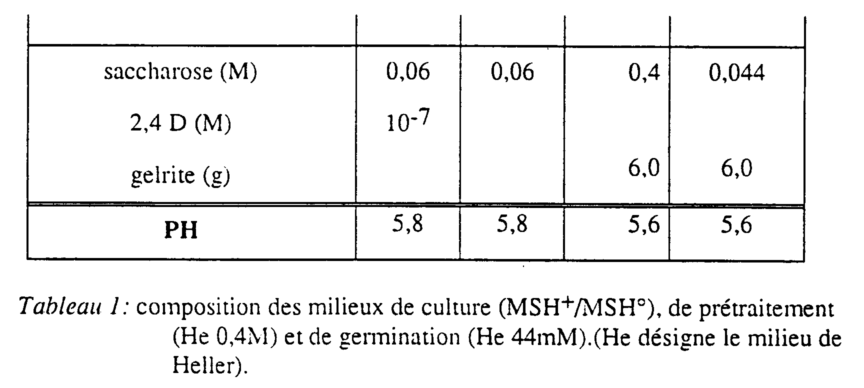

Pour obtenir des embryons, après 12 jours de culture sur milieu MSH⁺, la suspension cellulaire est filtrée une première fois sur toile à bluter de 100 µ de vide de maille. Le filtrat est repris et passé sur une toile à bluter de 60 µ de vide de maille. La fraction retenue par le filtre est rincée avec du milieu MSH°, de composition indiquée au tableau 1 ci-après, caractérisé par l'absence d'auxine puis reprise dans 45 ml de milieu MSH°, et centrifugée 5 minutes à 300 g (centrifugeuse Jouan C312). Le surnageant est ensuite prélevé et les amas cellulaires repris à nouveau dans 45 ml de MSH° neuf. Deux autres centrifugations successives sont ainsi réalisées.

On prélève ensuite 1 ml de suspension constituée du culot cellulaire repris dans 10 ml de MSH° que l'on centrifuge 5 min à 900 g ce qui permet d'évaluer la quantité de tissus dans un millilitre de suspension. Le matériel cellulaire est ensuite mis en suspension :
- soit dans du milieu MSH°, dans des Erlenmeyers de 1 litre contenant 500 ml de milieu, sur agitateur orbital tournant à 100 tours.min⁻¹, lumière et photopériode identiques aux conditions précédentes:
- soit dans du milieu MSH° contenant 0,02% de charbon actif(CA) (Merck), dans des Erlenmeyers de 500 ml contenant 250 ml de milieu, sur agitateur orbital tournant à 75 tours.min⁻¹, à l'obscurité.

La densité d'inoculation est de 1 g de biomasse par litre. Le matériel cellulaire est maintenu dans les conditions d'entretien de la suspension pendant les 12 jours nécessaires à l'obtention des embryons.

La récolte des embryons s'effectue après 12 jours de culture sur milieu MSH°. La suspension d'embryons est filtrée sur toile à bluter de 400 µ de vide de maille (Scrynel). La fraction retenue par le filtre est séparée du filtre par rinçage avec du milieu MSH° et déposée dans une boite de Petri de 9 cm de diamètre (Plastiques Gosselin; France: réf BP 90). Les embryons dont la taille est comprise entre 500 et 1500 µ sont prélevés à la pipette Pasteur sous la loupe binoculaire.

### EXEMPLE 2 : enrobage et pelliculage

a) Des supports, de volume unitaire d'environ 0,5cm3, constitués par des morceaux de filtre en acétate de cellulose, de poids unitaire inférieur à 200 mg, sont trempés dans le mélange de revêtement constitué de gélifiant (Carraghénane type I 0,5% et CaCl₂ 50 mM), d'un épaississant (Natrosol HHR250:1,5%) et d'une charge d'argile(Argirec B22: 20%). On obtient 100 ml de ce revêtement par solubilisation de 0,5g de Carraghénane type I dans de l'eau chaude, puis addition à la solution de 1,5 g de Natrosol HHR 250 et 730 mg de CaCl₂,2H₂O à chaud et addition à la solution de 25 a d'Argirec, sous forte agitation obtenue à l'aide d'un mélangeur Waring blendor.
   Les filtres cellulosiques sont ensuite mis à sécher.
   On introduit, avec l'aide dune pipette ou d'une pince , un embryon dans chacun des enrobages et par la partie de leur surface laissée libre.
b) On prépare une composition contenant respectivement 5% de gélatine, 10% de charge d'argile (Argirec B22, 1% d'un épaississant (Natrosol)) dans de l'eau distillée ou dans du milieu de culture. On soumet la solution chaude à une forte agitation pour former des bulles d'air, qui sont stabilisées par la présence de l'épaissant. On dépose, sur un support non adhérant, cette composition par aération sous forme de gouttes à une température où la gélatine n'est pas encore gélifiée(30 à 35°C). La gélatine, en se refroidissant, fige la structure bulleuse. On renverse la structure hémisphérique ainsi obtenue sur sa face convexe et on dépose l'embryon sur la face plane de la demi-sphère. Eventuellement on peut enfin coller une seconde demi-sphère sur la première en humectant les surfaces de contact.

On applique ensuite, sur une partie des embryons enrobés une ou deux couches d'un pelliculage externe sous forme d'une solution de chlorure de polyvinyle(3,33%), d'acétate de polyvinyle(3,33%) et de bentone SD1(3,33%) dans du tétrahydrofuranne. Une seconde formulation contenant 5% de chacun des mêmes constituants est ensuite également appliquée.

Pour être sûr de travailler en conditions stériles, on soumet, avant sa fixation sur le support, l'enrobage à un passage en autoclave, soit avant soit entre les deux pelliculages. Ce traitement assure en outre l'étanchéification complète des enrobages.

### EXEMPLE 3: Dessiccation

Le prétraitement conférant aux embryons l'aptitude à la dessiccation peut être effectué sur milieu gélosé. Dans ce cas, les embryons sont placés par lots de 25 sur papiers filtres (Whatman 1820025, Maidstone, Angleterre) et déposé dans des boites de Petri de 6 cm de diamètre (Caric: France) contenant du milieu de Heller 0.4M (composition of tableau 1) additionnés de 6 g/l de Phytagel (Sygma). Les boites sont scellées avec du film alimentaire "Scell'o'frais" puis mises en chambre froide 7 jours a' 4°C, a' l'obscurité.

Le prétraitement petit être effectué en milieu liquide à température ambiante. Le milieu MSH° de la phase embryogène est substitué par du milieu He 0,4M liquide. La suspension est ainsi mise en agitation à 100 tours.min⁻¹, à 4°C (4 jours) ou 25°C (3 jours), à l'obscurité.

Les embryons ayant subit le prétraitement sont ensuite placés dans des enceintes(dessiccateurs) où l'humidité relative est maintenue à 45% par une solution sursaturée de carbonate de potassium (Merck). La solution est constitué par un mélange de 40g de sels pour 16 ml d'eau. Les papiers filtres portant les embryons sont placés dans des boites de Petri vides de 6 cm de diamètre. Les boites sont ensuite mises en dessiccateurs. Les dessiccateurs sont scellés par un film alimentaire "Scell'o'frais" puis placés à l'obscurité pendant des temps variables( de 15 à 50 jours) à 4°C, de manière que la teneur en eau finale des embryons soit inférieure à 0,40 g par gramme de poids sec.

### EXEMPLE 4: Germination

Les embryons sont mis en germination par transfert du papier filtre les portant sur milieu de Heller 44 mM (composition of tableau 1), en boites de Petri de 6 cm de diamètre, au sortir du traitement préalable à la dessiccation ou après différentes dates de dessiccation. Les boites sont placées en chambre de culture à 25°C, sous une photopériode de 16 heures de jour et 8 heures de nuit et un éclairement de 55 µE.m⁻².s⁻¹. Les jeunes plantes atteignant une taille d'environ 1 cm sont transferrées en boites Plancton (Flow laboratories; USA) sur milieu de Heller 44 mM à raison de 30 jeunes plantes par boite.

Dans tous les exemples suivants, les observations sont établies à partir des paramètres suivants:
Les taux de survie et de germination des embryons placés sur milieu de Heller 44 mM sont mesurés quelques jours après leur transfert et définis comme suit:
- Taux de survie : nombre d'embryons présentant une évolution soit au niveau de la racine ou de l'apex rapporté au nombre total d'embryons.
- Taux de germination : nombre d'embryons présentant un apex chlorophyllien et une racine rapporté au nombre total d'embryons.

Le taux de conversion est évalué deux mois environ après le passage en boites Plancton. Il est défini par:
- Taux de conversion : nombre d'embryons présentant la première paire de feuilles vraies rapporté au nombre total d'embryons.

4A) "Embryons + enrobages sans dessiccation":
   Les embryons sont obtenus comme à l'exemple 1 (la sur milieu MSH° et les enrobages et pelliculages comme à l'exemple 2a);
4B) "Embryons désséchés + enrobage"
   Les embryons sont obtenus comme à l'exemple 1( sur milieu MSH°) puis desséchés comme à l'exemple 3: les enrobages et pelliculages sont obtenus comme à l'exemple 2:
4C) "Embryons enrobés désséchés"
   Les embryons sont obtenus comme à l'exemple 1 (sur milieu MSH°), puis enrobés comme à l'exemple 2a), Les embryons enrobés sont desséchés comme à l'exemple 3.

Les résultats relatifs aux embryons obtenus aux exemples 4A) à C) figurent dans le tableau 2 ci-après avec comparaison des taux respectifs de survie, de germination, de conversion et le cas échéant le taux de sortie des racines de l'enrobage, par rapport aux embryons nus, cultivés sur milieu sans charbon actif, non déshydratés (E.N.1) et déhydratés(E.N.2).

| EXEMPLE N° | Taux de survie | Taux de germination | Taux de conversion | Taux de sortie des racines |
|---|---|---|---|---|
| EN 1 | 98 | 98 | 77 | - |
| EN 2 | 86 | 86 | 70 | - |
| 4A | 95 | 95 | 45 | 65 |
| 4B | 100 | 100 | 95 | 95 |
| 4C | 100 | 85 | 70 | - |

Ces résultats font apparaître clairement que :
1) les embryons enrobés non désséchés (4A) présentent un excellent taux de survie et de germination:
2) les embryons enrobés désséchést (4C) présentent un excellent taux de survie et de germination avec un taux de conversion satisfaisant:
3) les embryons désséchés puis enrobés (4B) présentent non seulement un excellent taux de survie et de germination mais aussi un excellent taux de conversion et un taux de sortie des racines comparable au taux de conversion, ce qui prouve l'excellente aptitude à la levée des tissus méristématiques enrobés selon l'invention.

### Exemple 5: Essai de germination avec des graines enrobées:

a) Des graines de chicorée scarole de la société Clause sont mises à germées aseptiquement. Les graines sont désinfectées par passage deux heures dans une solution d'hypochlorite de calcium à 35g/l sous vide. Après trois rinçages, les graines sont placées en tube à essai contenant 10 ml de milieu de culture de Heller 44mM décrite ci-dessus, à raison d'une graine par tube, les graines étant préalablement enrobées ou non.
b) les graines sont placées dans des enrobages tels que décrits à l'exemple 2 a), après que l'enrobage ait été imprégné de 280µl de milieu de culture de Heller. Deux répétitions sont réalisées à raison de 10 graines par traitement et par répétition.

Dans ces conditions on obtient, 17 jours après le semis, les résultats suivants:
NT: graines témoins non enrobées
5: graines enrobées

| EXEMPLE N° | Taux de germination % | Taux de conversion % | Taux de sortie des racines % | Poids frais moyen des plantes(mg) |
|---|---|---|---|---|
| NT | 92 | 92 | 92 | 162 |
| 5 | 73 | 73 | 73 | 102 |

Ces résultats font apparaître clairement que les graines enrobées (5) présentent non seulement un taux de germination mais aussi un taux de conversion satisfaisant par rapport aux plants semés avec un taux de sortie des racines remarquable par rapport aux plants germés, ce qui prouve l'excellente aptitude à la levée des graines enrobées selon l'invention.

## Revendications

1. Semence artificielle comprenant un tissu méristématique enrobé, laquelle comprend un tissu méristématique, sec ou séchable, et un support solide sec ou séchable capable d'absorber un milieu nutritif, en contact avec le tissu méristématique, le recouvrant partiellement, de sorte que le tissu méristématique reste en contact avec un volume d'air, et ne faisant pas obstacle à la germination, caractérisée en ce que le support comporte une cavité de volume supérieur à celui du tissu méristématique.

2. Semence selon la revendication 1, caractérisée en ce que le tissu méristématique est sec.

3. Semence selon la revendication 1, caractérisée en ce que le tissu méristématique est séchable ou hydraté .

4. Semence selon l'une des revendications 1 à 3, caractérisée en ce que le support solide est sec.

5. Semence selon l'une des revendications 1 à 3, caractérisée en ce que le support solide est séchable ou hydraté.

6. Semence selon l'une des revendications 1 à 3, caractérisée en ce que le tissu méristématique est inséré dans un support solide fibreux.

7. Semence selon la revendication 6, caractérisée en ce que le support solide fibreux est un filtre en cellulose.

8. Semence selon l'une des revendications 1 à 7, caractérisée en ce que le support solide comprend un liant.

9. Semence selon l'une des revendications 1 à 8, caractérisée en ce que le support solide comprend un épaississant.

10. Semence selon l'une des revendications 1 à 9, caractérisée en ce qu'elle comprend en outre, autour du support, au moins une couche de pelliculage le recouvrant partiellement, de sorte que le tissu méristématique reste en contact avec un volume d'air.

11. Semence selon la revendication 10, caractérisée en ce que, entre le support et la couche de pelliculage, se trouve une couche intermédiaire d'enrobage.

12. Semence selon la revendication 10, caractérisée en ce que l'enrobage comprend un liant.

13. Semence selon la revendication 10, caractérisée en ce que l'enrobage comprend un épaississant.

14. Procédé de fabrication d'une semence artificielle selon l'une des revendications 1 à 13, dans lequel on cultive une suspension cellulaire végétale jusqu'au stade embryonnaire créant un tissu méristématique, et
on fixe le tissu méristématique à un support solide sec ou séchable, capable d'absorber un milieu nutritif et ne faisant pas obstacle à la germination, de sorte que le support le recouvre partiellement et que le tissu méristématique reste en contact avec un volume d'air, caractérisé en ce que le support comporte une cavité de volume supérieur à celui du tissu méristématique.

15. Procédé selon la revendication 14, caractérisé en ce que, avant ou après la mise en place du tissu méristématique, on dépose sur le support au moins une couche de pelliculage le recouvrant partiellement, de sorte que le tissu méristématique, une fois fixé sur le support reste en contact avec un volume d'air.

16. Procédé selon l'une des revendications 14 et 15, caractérisé en ce que, avant ou après la mise en place du support, le tissu méristématique est desséché.

17. Utilisation d'une semence artificielle selon l'une des revendications 1 à 7, par semis ou mise en culture.

## Claims

1. Artificial seed containing a coated meristematic tissue, which comprises a dry or driable meristematic tissue, and a dry or driable solid support capable of absorbing a nutrient medium, which is in contact with the meristematic tissue, partly covering it in such a way that the meristematic tissue remains in contact with a volume of air, and which does not hinder germination, characterized in that the support includes a cavity whose volume is greater than that of the meristematic tissue.

2. Seed according to Claim 1, characterized in that the meristematic tissue is dry.

3. Seed according to Claim 1, characterized in that the meristematic tissue is driable or hydrated.

4. Seed according to one of Claims 1 to 3, characterized in that the solid support is dry.

5. Seed according to one of Claims 1 to 3, characterized in that the solid support is driable or hydrated.

6. Seed according to one of Claims 1 to 3, characterized in that the meristematic tissue is inserted into a fibrous solid support.

7. Seed according to Claim 6, characterized in that the solid fibrous support is a cellulose filter.

8. Seed according to one of Claims 1 to 7, characterized in that the solid support contains a binder.

9. Seed according to one of Claims 1 to 8, characterized in that the solid support contains a thickener.

10. Seed according to one of Claims 1 to 9, characterized in that it furthermore contains, around the support, at least one layer of skin formation which covers it partly in such a way that the meristematic tissue remains in contact with a volume of air.

11. Seed according to Claim 10, characterized in that there is an intermediate layer of coating between the support and the layer of skin formation.

12. Seed according to Claim 10, characterized in that the coating contains a binder.

13. Seed according to Claim 10, characterized in that the coating contains a thickener.

14. Process for the preparation of an artificial seed according to one of Claims 1 to 13, in which a plant cell suspension is cultured up to the embryo stage, creating a meristematic tissue, and the meristematic tissue is attached to a dry or driable solid support which is capable of absorbing a nutrient medium and does not hinder germination, in such a way that the support covers it partly and that the meristematic tissue remains in contact with a volume of air, characterized in that the support includes a cavity whose volume is greater than that of the meristematic tissue.

15. Process according to Claim 14, characterized in that at least one layer of skin formation which partly covers the support is deposited on it before or after placing the meristematic tissue in its location, in such a way that the meristematic tissue remains in contact with a volume of air once it is attached to the support.

16. Process according to one of Claims 14 and 15, characterized in that the meristematic tissue is dried before or after the support is placed in its location.

17. Use of an artificial seed according to one of Claims 1 to 7 by sowing or culturing.

## Patentansprüche

1. Künstliches, ein umhülltes meristematisches Gewebe aufweisendes Saatgut, das ein trockenes oder trockenbares meristematisches Gewebe und einen trockenen oder trockenbaren Träger, der ein Nährmedium zu absorbieren vermag, im Kontakt mit dem meristematischen Gewebe umfaßt, der das meristematische Gewebe teilweise bedeckt, so daß das meristematische Gewebe im Kontakt mit einem Luftvolumen bleibt, und der kein Hindernis für die Keimung darstellt, dadurch gekennzeichnet, daß der Träger ein Hohlraumvolumen aufweist, das größer ist als das Volumen des meristematischen Gewebes.

2. Saatgut nach Anspruch 1, dadurch gekennzeichnet, daß das meristematische Gewebe trocken ist.

3. Saatgut nach Anspruch 1, dadurch gekennzeichnet, daß das meristematische Gewebe trockenbar oder hydratisiert ist.

4. Saatgut nach einem der Ansprüche 1 bis 3, dadurch gekennzeichnet, daß der feste Träger trocken ist.

5. Saatgut nach einem der Ansprüche 1 bis 3, dadurch gekennzeichnet, daß der feste Träger trockenbar oder hydratisiert ist.

6. Saatgut nach einem der Ansprüche 1 bis 3, dadurch gekennzeichnet, daß das meristematische Gewebe in einen faserigen festen Träger eingebracht ist.

7. Saatgut nach Anspruch 6, dadurch gekennzeichnet, daß der faserige feste Träger ein Cellulosefilter ist.

8. Saatgut nach einem der Ansprüche 1 bis 7, dadurch gekennzeichnet, daß der feste Träger ein Bindemittel enthält.

9. Saatgut nach einem der Ansprüche 1 bis 8, dadurch gekennzeichnet, daß der feste Träger ein Verdickungsmittel enthält.

10. Saatgut nach einem der Ansprüche 1 bis 9, dadurch gekennzeichnet, daß es außerdem um den Träger herum eine Überzugsschicht aufweist, die den Träger teilweise bedeckt, so daß das meristematische Gewebe im Kontakt mit einem Luftvolumen bleibt.

11. Saatgut nach Anspruch 10, dadurch gekennzeichnet, daß zwischen dem Träger und der Überzugsschicht eine Umhüllungszwischenschicht vorhanden ist.

12. Saatgut nach Anspruch 10, dadurch gekennzeichnet, daß die Umhüllung ein Bindemittel enthält.

13. Saatgut nach Anspruch 10, dadurch gekennzeichnet, daß die Umhüllung ein Verdickungsmittel enthält.

14. Verfahren zur Herstellung eines künstlichen Saatguts nach einem der Ansprüche 1 bis 13, bei dem eine Suspension pflanzlicher Zellen bis zum Embryonalstadium kultiviert wird, in dem ein meristematisches Gewebe erzeugt wird, und bei dem das meristematische Gewebe so an einem trockenen oder trockenbaren Träger, der ein Nährmedium zu absorbieren vermag und kein Hindernis für die Keimung darstellt, fixiert wird, daß der Träger das meristematische Gewebe teilweise bedeckt und das meristematische Gewebe im Kontakt mit einem Luftvolumen bleibt, dadurch gekennzeichnet, daß der Träger ein Hohlraumvolumen aufweist, das größer ist als das Volumen des meristematischen Gewebes.

15. Verfahren nach Anspruch 14, dadurch gekennzeichnet, daß vor oder nach dem Anordnen des meristematischen Gewebes mindestens eine Überzugsschicht auf den Träger aufgebracht wird, die den Träger teilweise bedeckt, so daß das meristematische Gewebe, wenn es an dem Träger fixiert ist, im Kontakt mit einem Luftvolumen bleibt.

16. Verfahren nach einem der Ansprüche 14 und 15, dadurch gekennzeichnet, daß das meristematische Gewebe vor oder nach dem Anordnen des Trägers getrocknet wird.

17. Verwendung eines künstlichen Saatguts nach einem der Ansprüche 1 bis 7 zum Aussäen oder zum Bebauen.
